# EUROPEAN PATENT SPECIFICATION

(11) **EP 0 753 253 B1**
(45) Date of publication and mention of the grant of the patent: **10.10.2001**
(21) Application number: 96111198.6
(22) Date of filing: 11.07.1996
(51) Int. Cl.: A01M 1/20, A01M 13/00

(54) **Sustained release pheromone-containing preparations**
Pheromonen enthaltendes Präparät mit verlängerter Freisetzung
Préparations contenant des phéromones, à diffusion prolongée

(30) Priority: 13.07.1995 JP 17753895
(43) Date of publication of application: 15.01.1997
(73) Proprietor: Shin-Etsu Chemical Co., Ltd., Chiyoda-ku Tokyo 100 (JP)
(72) Inventor: Suzuki, Hiroshi, c/o Shin-Etsu Chem. Co., Ltd., Nakakubiki-gun, Niigata 942-01 (JP); Aiba, Noboru, c/o Shin-Etsu Chem. Co., Ltd., Nakakubiki-gun, Niigata 942-01 (JP); Saguchi, Ryuichi, c/o Shin-Etsu Chem. Co., Ltd., Nakakubiki-gun, Niigata 942-01 (JP); Ogawa, Kinya, c/o Shin-Etsu Chem. Co., Ltd., Tokyo 100 (JP)
(74) Representative: Tiedtke, Harro, Dipl.-Ing.

(56) References cited:
- EP-A- 0 342 126
- EP-A- 0 683 977
- US-A- 5 219 121
- PATENT ABSTRACTS OF JAPAN vol. 014, no. 294 (C-0732), 26 June 1990 & JP 02 096502 A (SHIN ETSU CHEM CO LTD), 9 April 1990,
- PATENT ABSTRACTS OF JAPAN vol. 013, no. 021 (C-560), 18 January 1989 & JP 63 225303 A (SHIN ETSU CHEM CO LTD), 20 September 1988,

## Description

The present invention relates to a sustained release pheromone-containing preparation which is used for controlling the proliferation of harmful insects.

There has been required for the development of a pharmaceutical or agricultural preparation which allows gradual release of a volatile drug to thus ensure a long lasting effect thereof. The communication-disturbing method should satisfy the foregoing requirement, this method comprising controlling the breeding of harmful insects through release of, for instance, a sex pheromone at a predetermined concentration over a long time period. The term "communication-disturbing method" herein means a method which comprises distributing a sex pheromone in a field at a concentration substantially higher than that released from a harmful insect to thus lower the communication ability of the insect such as an ability of male or female insects to recognize the individual opposite sex and to confirm the positions thereof, and to thereby inhibit copulation of the insects.

As an example of such a sustained release pheromone-containing preparation, there has been known a preparation packed in a bag-like container. For instance, Japanese Patent Application Publication No. 6-88886 and Japanese Patent Provisional Publication Nos. 2-49702 (corresponding to EP-A-0 342 126) and 2-96502 disclose preparations each of which comprises a liquid sex pheromone accommodated in a bag made of a film having appropriate barrier properties to the sex pheromone. This bag-like sustained release pheromone preparation can release the pheromone through penetration of the liquid sex pheromone accommodated in the bag into the film and subsequent evaporation thereof through the outer wall of the film. The pheromone preparation of this type permits the release of a uniform amount of the pheromone over a long time period since the liquid sex pheromone is always in contact with the inner wall of the bag.

However, the bag-like sustained release pheromone preparation suffers from a problem in that if the amount of the liquid pheromone remaining in the bag decreases, the contacting surface area between the liquid sex pheromone and the inner surface of the bag is accordingly reduced, this is because the contact angle between the inner surface of the bag and the pheromone becomes not more than 90° due to the surface tension of the latter and the liquid pheromone makes a backward movement from the film. For this reason, the amount of the sex pheromone released from the preparation is gradually reduced as the amount of the pheromone remaining in the bag decreases and therefore, it has been impossible to ensure any uniform release of the pheromone till the liquid pheromone is completely exhausted.

The present invention has been developed for solving the foregoing problems of the conventional techniques and accordingly, it is an object of the present invention to provide a sustained release pheromone-containing preparation which ensures uniform release of the sex pheromone till the pheromone present therein is completely exhausted, irrespective of the amount of the pheromone remaining in a bag.

The foregoing object of the present invention can effectively be accomplished by providing a sustained release pheromone-containing preparation which comprises a bag formed from a permeable film provided with a highly wettable film on the inner surface thereof and a liquid sex pheromone accommodated in the bag. The highly wettable film continuously ensures contact between the permeable film and the liquid sex pheromone.

Fig. 1 is a schematic cross sectional view of an embodiment of the, sustained release pheromone-containing preparation according to the present invention.

Fig. 2 is a schematic cross sectional view of another embodiment of the sustained release pheromone-containing preparation according to the present invention.

Fig. 3 is a diagram showing the relation between the elapsed time (day) and the amount of the sex pheromone released from the sustained release preparations.

The present invention will hereinafter be described in more detail with reference to specific embodiments and the accompanying drawings.

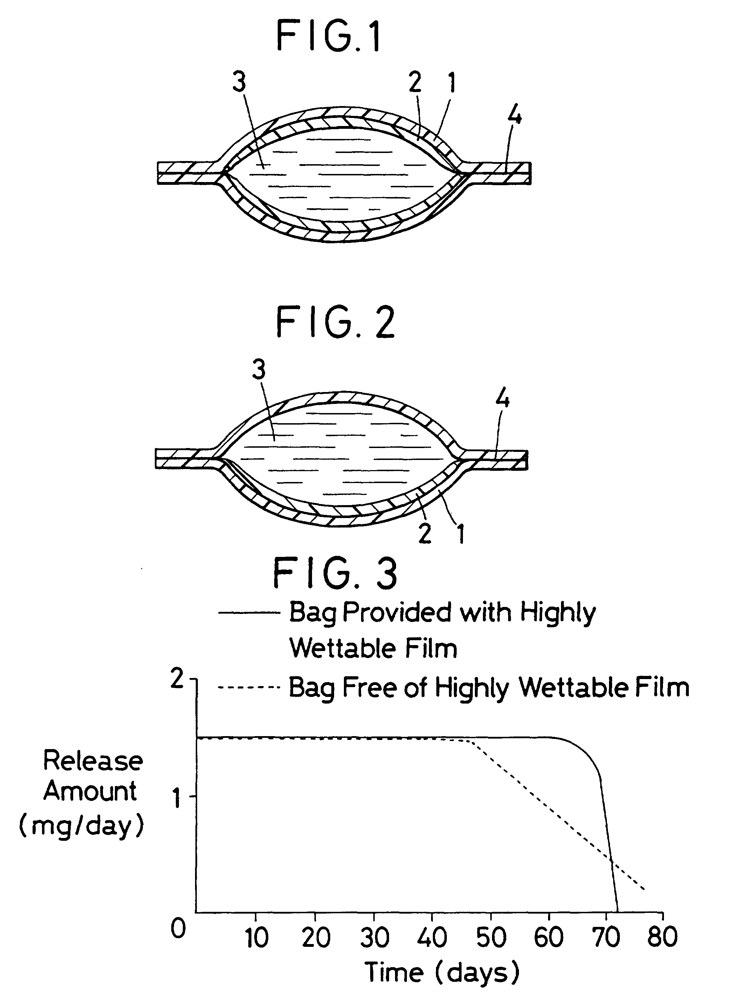

As shown in Fig. 1, the sustained release pheromone-containing preparation according to the present invention comprises a bag formed from a permeable film 1 provided with a highly wettable film 2 on the inner surface thereof and a liquid sex pheromone 3 accommodated in the bag.

The highly wettable film 2 is preferably set up on one or both sides of the inner wall of the bag as shown in Fig. 1 or 2.

The highly wettable film 2 is preferably selected from the group consisting of nonwoven fabrics of polypropylenes, polyethylenes, polyesters and nylons; nonwoven fabrics of polyolefin species consisting of binary materials which comprise a core material of a polyester or nylon; and porous films of polyesters, nylons, polysulfones, polyethylenes and polypropylenes, which may be used alone or in any combination.

According to the sustained release pheromone-containing preparation having the aforementioned construction, the highly wettable film 2 set up on the inner wall of the bag is always wetted with the liquid sex pheromone 3 due to the capillary action thereof and this, in turn, always ensures the contact between the permeable film 1 constituting the bag and the liquid sex pheromone 3, as will be seen from Fig. 1.

The present invention will hereinafter be described in more detail with reference to the following non-limitative working Examples and Comparative Examples.

Fig. 1 is a schematic cross sectional view of an embodiment of the sustained release pheromone-containing preparation according to the present invention. As seen from this figure, the preparation according to this embodiment comprises a bag made from a permeable film 1 provided with a highly wettable film 2 on both sides of the inner wall of the bag (i.e., the whole inner wall of the bag) and a liquid sex pheromone 3 accommodated in the bag.

The sex pheromone 3 is absorbed by the highly wettable film 2 to thus wet the film 2 and subsequently penetrates into the permeable film 1. The liquid sex pheromone 3 penetrated into the film 1 is evaporated through the outer surface of the bag and thus released into the open air.

The sustained release pheromone-containing preparation which is provided with the highly wettable film 2 on both sides of the inner wall of the bag, as shown in Fig. 1, is prepared as follows. Highly wettable films 2 are adhered to one side each of two permeable films 1 except for the portion 4 at which these permeable films 1 are ultimately sealed to give two sheets provided with the wettable film 2, followed by putting these sheets in layers in such a manner that the highly wettable films 2 face each other and closing the sheets at three sides through heat sealing to give a bag whose one side is opened. The liquid sex pheromone 3 is injected into the bag through the opening and then the opened side is closed through heat sealing.

Alternatively, the sustained release pheromone-containing preparation of the invention may comprise the highly wettable film 2 only on one side of the inner wall of the bag (i.e., a part of the inner surface of the bag) as shown in Fig. 2.

Moreover, the shape of the bag may be a four side-sealed bag, a three side-sealed bag, pillow-shaped packaging bag or a stick-like packaging bag.

More specifically, there was used, as the permeable film 1, a double-layered film comprising a low density polyethylene (LDPE) film having a thickness of 40 µm and a biaxially oriented polypropylene (OPP) film having a thickness of 30 µm. Then two three-layered laminate sheets each was prepared by putting a highly wettable film 2 of a polypropylene nonwoven fabric having a surface area of 6 cm² and a thickness of 75 µm on the LDPE side of the double-layered film except for the portion 4 at which the permeable film 1 was ultimately sealed. A bag was prepared from these two laminate sheets, followed by injecting, into the bag, 100 mg of a liquid sex pheromone 3, i.e., Z-11-tetradecenyl acetate as a sex pheromone component of oriental tea tortrix (Homona magnanima Diakonoff) to give a sustained release pheromone-containing preparation wherein highly wettable films 2 were set up on both sides (having an overall surface area of 12 cm²) of the inner wall of the bag.

The resulting pheromone-containing preparation was allowed to stand under the conditions of a temperature of 30 °C and a wind velocity of 0.5 m/sec to determine the change, with time, in the amount of Z-11-tetradecenyl acetate released from the preparation. The results thus obtained are plotted on Fig. 3 as a solid line (specified as "Bag Provided with Highly Wettable Film"). The data plotted on Fig. 3 clearly indicate that the preparation could maintain a uniform released amount of the pheromone on the order of 1.5 mg/day over not less than 60 days.

By way of comparison, a comparative sustained release pheromone-containing preparation was also prepared by producing a bag from only two double-layered sheets comprising an LDPE film having a thickness of 40 m and an OPP film having a thickness of 30 µm without using any highly wettable film 2, followed by injecting 100 mg of Z-11-tetradecenyl acetate as the liquid sex pheromone 3. The comparative preparation was likewise be allowed to stand under the conditions of a temperature of 30 °C and a wind velocity of 0.5 m/sec to determine the change, with time, in the amount of Z-11-tetradecenyl acetate released from the resulting preparation. The results thus obtained are plotted on Fig. 3 as a chain line (specified as "Bag Free of Highly Wettable Film"). As seen from Fig. 3, the comparative preparation maintained a uniform release rate of 1.5 mg/day over 45 days, but the release rate thereof was then gradually reduced.

As has been discussed above in detail, the sustained release pheromonecontaining preparation according to the present invention comprises a bag formed from a permeable film provided with a highly wettable film on a part or the whole inner surface thereof and a liquid sex pheromone accommodated in the bag and can maintain a uniform release rate of the sex pheromone over a long time period and can ensure a uniform release rate of the sex pheromone until the pheromone is completely exhausted, irrespective of the amount of the pheromone remaining in the bag.

## Claims

1. A sustained release pheromone-containing preparation comprising a bag formed from a permeable film (1) provided with a film (2) on the inner surface thereof and a liquid sex pheromone (3) accommodated in the bag,
**characterized in that**
said film (2) is highly wettable so as to continuously ensuring contact between said permeable film (1) and said liquid sex pheromone (3).

2. The sustained release pheromone-containing preparation of claim 1 wherein the highly wettable film (2) is at least one member selected from the group consisting of nonwoven fabrics of polypropylenes, polyethylenes, polyesters and nylons; nonwoven fabrics of polyolefin species consisting of binary materials which comprise a core material of a polyester or nylon; and porous films of polyesters, nylons, polysulfones, polyethylenes and polypropylenes.

3. The sustained release pheromone-containing preparation of claim 1 or 2, wherein the highly wettable film (2) is set up on a part or the whole inner surface of the bag.

## Patentansprüche

1. Pheromon-haltiges Präparat mit Depotwirkung, das folgendes umfasst:
einen aus einem durchlässigen Film (1) gebildeten Beutel, der mit einem Film (2) auf dessen innerer Oberfläche ausgestattet ist, und
ein in dem Beutel untergebrachtes, flüssiges Sexual-Pheromon (3),
**dadurch gekennzeichnet, dass**
der Film (2) hoch-benetzbar ist, um so einen kontinuierlichen Kontakt zwischen dem durchlässigen Film (1) und dem flüssigen Sexual-Pheromon (3) sicherzustellen.

2. Pheromon-haltiges Präparat mit Depotwirkung gemäß Anspruch 1, wobei der hoch-benetzbare Film (2) wenigstens ein Element ist, das aus der folgenden Gruppe ausgewählt ist:
nicht-wollene Gewebe aus Polypropylenen, Polyethylenen, Polyestern und Nylons;
nicht-wollene Gewebe aus Polyolefin-Arten, die aus binären Materialien bestehen, welche ein Kernmaterial aus einem Polyester oder Nylon einschließen; und
poröse Filme aus Polyestern, Nylons, Polysulfonen, Polyethylenen und Polypropylenen.

3. Pheromon-haltiges Präparat mit Depotwirkung gemäß Anspruch 1 oder 2, wobei der hoch-benetzbare Film (2) auf einem Teil oder der gesamten inneren Oberfläche des Beutels angebracht ist.

## Revendications

1. Préparation contenant une phéromone à libération prolongée comprenant une poche constituée par un film perméable (1) muni d'un film (2) sur sa surface interne et une phéromone sexuelle liquide (3) contenue dans la poche, **caractérisée en ce que**
ledit film (2) est hautement mouillable de manière à assurer continuellement un contact entre ledit film perméable (1) et ladite phéromone sexuelle liquide (3).

2. Préparation contenant une phéromone à libération prolongée selon la revendication 1, où le film hautement mouillable (2) est au moins un membre choisi dans le groupe consistant en les étoffes non tissées de polypropylènes, de polyéthylènes, de polyesters et de Nylons ; les étoffes non tissées d'espèces de polyoléfines consistant en matériaux binaires qui comprennent un matériau formant noyau en un polyester ou Nylon ; et les films poreux de polyesters, de Nylons, de polysulfones, de polyéthylènes et de polypropylènes.

3. Préparation contenant une phéromone à libération prolongée selon la revendication 1 ou 2, où le film hautement mouillable (2) est placé sur une partie ou sur la totalité de la surface interne de la poche.
